(19) Europäisches Patentamt
European Patent Office
Office européen des brevets

(11) Publication number: **0 157 365**
**B1**

(12) **EUROPEAN PATENT SPECIFICATION**

(45) Date of publication of patent specification: **13.06.90**

(51) Int. Cl.$^5$: **C 07 B 57/00**

(21) Application number: **85103702.8**

(22) Date of filing: **27.03.85**

(54) **Separation agent comprising polysaccharide carbamate.**

(30) Priority: **02.04.84 JP 65322/84**

(43) Date of publication of application:
**09.10.85 Bulletin 85/41**

(45) Publication of the grant of the patent:
**13.06.90 Bulletin 90/24**

(84) Designated Contracting States:
**BE CH DE FR GB IT LI NL**

(56) References cited:
**US-A-2 668 168**

**BIOCHEMISTRY, vol. 12, no. 10, 1973, pages 1985-1990; P.J. CASHION et al.: "Use of trityl- and alpha-naphthylcarbamoylcellulose derivatives in oligonucleotide synthesis"**

(73) Proprietor: **DAICEL CHEMICAL INDUSTRIES, LTD.**
**No. 1-Banchi, Teppo-cho**
**Sakai-shi Osaka-fu 590 (JP)**

(72) Inventor: **Okamoto, Yoshio**
**24-11, Mukonoso-Higashi 1-chome**
**Amagasaki-shi Hyogo (JP)**
Inventor: **Hatada, Koichi**
**4-11, Asahigaoka 3-chome**
**Ideda-shi Osaka (JP)**

(74) Representative: **Patentanwälte Grünecker, Kinkeldey, Stockmair & Partner**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 157 365 B1**

Courier Press, Leamington Spa, England.

## Description

The invention relates to a method of separation of optical isomers, geometrical isomers or polymers having different molecular weight ranges from a mixture.

As is well-known, optical isomers usually exhibit different effects upon living organisms though they are chemically identical to each other. Therefore in order to improve the effectiveness of a medicine or an agricultural chemical per unit dose or to prevent side effects or damages caused by it in the fields of medicines, agricultural chemicals and biochemistry-related industries, the preparation of optically pure compounds is an important task.

Although a preferential crystallization method or a diastereomer method has heretofore been used to perform separation of optical isomers, these methods are applicable to rather limited kinds of compounds and frequently require a long time and much labor. Therefore, there has been a demand for a technique of effecting the optical resolution by a more practical chromatographic method.

Studies on the optical resolution by chromatography have long been made. However, it has been difficult to apply resolving agents developed heretofore satisfactorily to the optical resolution of all compounds, because there are various problems in that they have poor resolution efficiencies, that compounds to be resolved must have special functional groups, and that the resolving agents have poor stability.

Biochemistry, Vol. 12, No. 10, 1973, pages 1985 to 1990 discloses the use of trityl and α-naphthyl-carbamoyl-cellulose derivatives in oligonucleotide synthesis.

US—A—2668168 discloses cellulose trisalkylcarbamates having from 2 to 18 C-atoms in a straight chain.

EP—A—0156382 which is relevant under Article 54(3) and (4) EPC discloses a method of separating a chemical substance from a mixture by treating said mixture with a polysaccharide derivative containing a substituent of the formula

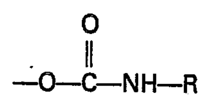

wherein R represents an atomic group having a nucleus comprising a conjugated π-bond system in which the number of bonds interposed between an atom contained therein and bonded with the amino group and an atom contained in the π-bond system and most distant from said atom is least 5 even in the shortest route.

It is therefore the object of the present invention to provide a method of separation of optical isomers, geometrical isomers or polymers having different molecular weight ranges by using a resolving agent which has resolution characteristics different from other resolving agents or has a higher discriminating power for optical isomers.

Said object is achieved by a method of separation of optical isomers, geometrical isomers or polymers having different molecular weight ranges from a mixture which comprises treating said mixture with a carbamate derivative of a polysaccharide selected from the group consisting of cellulose, amylose, chitosan, xylan, dextran and inulin, with the proviso that the polysaccharide does not contain a substituent of the formula

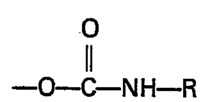
(I)

wherein R represents an atomic group having a nucleus comprising a conjugated π-bond system in which the number of bonds interposed between an atom contained therein and bonded with the amino group and an atom contained in the π-bond system and most distant from said atom is at least 5 even in the shortest route.

The polysaccharides used in the method of this invention are easily available as highly pure polysaccharides.

It is preferred that the number-average degree of polymerization (the average number of pyranose of furanose rings per molecule) of these polysaccharides is at least 5, preferably at least 10, and not higher than 500, because of easiness of handling, though there is no special upper limit.

The carbamoyl groups of the polysaccharide carbamate derivative used in the method of the present invention are N-substituted carbamoyl groups of 2 to 30, preferably 2 to 20, atoms and the substituent on the N atoms includes aromatic groups such as phenyl, α- or β-naphthyl or 4-nitrophenyl; heteroaromatic groups; substituted derivatives thereof; aliphatic (alicyclic) groups such as methyl; and substituted derivatives thereof.

In said carbamate derivatives, 30 to 100%, preferably 85 to 100%, on the average, of the total hydroxyl groups of the corresponding polysaccharides form urethane bonds together with said carbamoyl groups. Although the hydroxyl groups other than the above may be free hydroxyl groups, they may be esterified,

carbamoylated or etherified to an extent not detrimental to the isomer resolving power of said carbamate derivatives.

For the synthesis of said carbamate derivatives, a common reaction for forming a urethane from an alcohol and an isocyanate can be applied directly. For example, they can be obtained by reacting polysaccharides with the corresponding isocyanates by using a catalyst comprising a Lewis base such as a tertiary amine or a Lewis acid such as a tin compound.

When the resolving agent is used for resolving a compound or its optical isomers, it is a general practice to resort to chromatographic means such as gas chromatography, liquid chromatography, or thin layer chromatography, but it is also possible to perform membrane separation.

When the resolving agent used in the method of the present invention is applied to liquid chromatography, use is made of methods such as one in which the agent in the form of a powder is packed in a column, one in which the agent is applied to a capillary column as a coating, one in which the agent is formed into capillaries to utilize their inside walls, and one in which the agent is spun into fibers and a bundle of fibers is used as a column. Among these methods, the powder method is the most general.

When said resolving agent is formed into a powder, it is preferable to grind it or to form beads. Although the particle size varies with the size of a column or a plate used, it is 1 µm to 10 mm, preferably 1 µm to 300 µm, and it is preferable that the particles are porous.

In order to improve the pressure durability of the resolving agent, to prevent its swelling or shrinkage caused by solvent replacement, and to improve the number of theoretical plates, it is preferable to support said separation agent on a carrier. Although the suitable size of a carrier varies with the size of a column or a plate used, it is generally 1 µm to 10 mm, preferably 1 µm to 300 µm. The carrier is preferably porous, and the average pore diameter is 10 Å to 100 µm, preferably 50 Å to 50,000 Å. The amount of said resolving agent supported is 1 to 100 wt.%, preferably 5 to 50 wt.%, based on the carrier.

The method for supporting said resolving agent on a carrier may be a chemical or physical one. Examples of physical methods include one comprising dissolving said resolving agent in a solvent in which the agent is soluble, thoroughly mixing the solution with a carrier, and distilling off the solvent from the mixture by evacuation or passing an air stream with heating and one comprising dissolving said resolving agent in a solvent in which the agent is soluble, thoroughly mixing the solution with a carrier, and dispersing the mixture by agitation in a liquid incompatible with said solvent to diffuse said solvent into the liquid. When crystallizing said resolving agent supported on the carrier in this way, it is possible to treat it for example, by heating. It is also possible to modify the state of supporting and, in turn, its resolving power by swelling or dissolving said resolving agent in a small amount of a solvent and distilling off the solvent again.

The carriers which can be mentioned include porous organic and inorganic carriers, among which the latter are preferable. Examples of the porous organic carriers which can be suitably used include high-molecular substances such as polystyrene, polyacrylamide, and polyacrylate. Examples of the porous inorganic carriers which can be suitably used include synthetic and natural substances such as silica, alumina, magnesia, titanium oxide, glass, silicates, and kaolin, and it is possible to subject them to a surface treatment to improve their affinities for said resolving agent. Examples of the surface treatments include a silane treatment in which an organosilane is used and a surface treatment by plasma polymerization.

When said resolving agent is applied to the separation of compounds or the resolution of optical isomers, even resolving agents which are chemically the same frequently show different separation characteristics if they differ in physical properties such as molecular weight, crystallinity, and orientability. Therefore, when said resolving agent is applied according to any of the above-mentioned processes, it is possible to modify the separation characteristics so as to meet the intended use by selecting solvents used in the process, or performing heat-treatment or etching after the process, or physical treatment such as swelling with a liquid.

There is no particular limitation on developing solvents which are used in performing liquid or thin layer chromatography, except that those liquids which dissolve said resolving agent or react therewith must be excluded. There is no particular limitation on developing solvents except that reactive liquids must be excluded, when said resolving agent is bonded to a carrier by a chemical method, or it is insolubilized by crosslinking. Since the separation characteristics of compounds or optical isomers are, of course, affected by the developing solvent used, it is desirable to select a suitable developing solvent according to the purpose.

In performing thin layer chromatography, it is desirable to coat a support with a 0.1 to 100 mm-thick layer comprising said resolving agent in the form of particles of a diameter of 0.1 µm to 0.1 mm and, if necessary, a small amount of a binder.

When performing membrane separation, the resolving agent is used in the form of a hollow yarn or a film.

The resolving agent used in the method of the present invention comprising a carbamate derivative of a polysaccharide as an effective component is effective for the separation of a variety of compounds, and especially extremely effective for the resolution of optical isomers which are heretofore very difficultly separated. One of the optical isomers of a compound to be resolved will be adsorbed more strongly than the other on the resolving agent.

The present invention will now be described in detail with reference to examples. The definitions of the terms used in the examples are as follows:

$$\text{capacity ratio } (k') = \frac{(\text{retention volume of enantiomer}) - (\text{void volume})}{(\text{void volume})}$$

$$\text{separation factor } (\alpha) = \frac{\text{capacity ratio of enantiomer adsorbed more strongly}}{\text{capacity ratio of enantiomer adsorbed less strongly}}$$

$$\text{resolution } (Rs) = \frac{2 \times (\text{distance between peaks of more strongly adsorbed enantiomer and more weakly adsorbed enantiomer})}{\text{total of band widths of both peaks}}$$

Synthesis Example 1
(synthesis of cellulose trimethylcarbamate)

An excess (10 ml) of methyl isocyanate was added to a mixture (1 g:1.5 g:15 g: 1 g) of cellulose, LiCl, N,N-dimethylacetamide and pyridine, and the resulting mixture was kept at about 80°C to complete the reaction. The reaction product was precipitated from a large amount of methanol, and the precipitate was filtered through a glass filter, washed with methanol and dried in vacuo.

Elementary analysis

| | %C | %H | %N |
|---|---|---|---|
| Found: | 43.02 | 5.88 | 14.90 |
| Calculated: | 43.24 | 5.75 | 12.61 |

Synthesis Example 2
(synthesis of amylose trisphenylcarbamate)

1 g of amylose and 8 ml of phenyl isocyanate were added to 50 ml of pyridine, and the mixture was kept at about 110°C to complete the reaction.

The reaction product was precipitated from a large amount of methanol, and the precipitate was filtered through a glass filter, washed with methanol and dried in vacuo.

Elementary analysis

| | %C | %H | %N |
|---|---|---|---|
| Found: | 61.20 | 4.70 | 7.95 |
| Calculated: | 62.42 | 4.85 | 8.09 |

Synthesis Examples 3 through 6

Xylan, chitosan, dextran, or inulin was reacted in the same way as in Example 2.

Results of the elementary analysis of xylan bisphenylcarbamate (Synthesis Example 3)

| | %C | %H | %N |
|---|---|---|---|
| Found: | 59.07 | 5.20 | 6.88 |
| Calculated: | 61.62 | 4.90 | 7.56 |

Results of the elementary analysis of chitosan trisphenylcarbamate (Synthesis Example 4)

| | %C | %H | %N |
|---|---|---|---|
| Found: | 61.50 | 4.51 | 10.18 |
| Calculated: | 62.54 | 5.05 | 10.81 |

Results of the elementary analysis of dextran trisphenylcarbamate (Synthesis Example 5)

| | %C | %H | %N |
|---|---|---|---|
| Found: | 60.39 | 4.78 | 7.61 |
| Calculated: | (62.42) | (4.85) | (8.09) |

Results of the elementary analysis of inulin trisphenylcarbamate (Synthesis Example 6)

| | %C | %H | %N |
|---|---|---|---|
| Found: | 62.00 | 4.84 | 8.09 |
| Calculated: | 62.42 | 4.85 | 8.09 |

## Examples 1 through 6

A mixture (10 g:50 g:1 g:2 g) of silica gel (trademark LiChrospher® SI—4000 (a product of Merck)), benzene dried over metallic sodium, pyridine, and γ-aminopropyltriethoxysilane was reacted at 80°C for 16 h under a stream of nitrogen. The reaction mixture was poured into methanol, and the precipitate was filtered, washed with methanol and dried.

Elementary analysis: C: 0.23%, H: 0.07%, N: 0.09%.

750 mg of each of the polysaccharide derivatives obtained in Synthesis Examples 1 through 6 was dissolved in 10 ml of a solvent shown below, and the solution was mixed with the silane-treated silica gel obtained above. The solvent was distilled off to prepare a supported resolving agent.

| Example No. | Polysaccharide derivative | Solvent |
|---|---|---|
| 1. | cellulose trismethylcarbamate | N,N-dimethylacetamide |
| 2. | amylose trisphenylcarbamate | dioxane |
| 3. | chitosan trisphenylcarbamate | chloroform/formaldehyde (10:1.5) |
| 4. | xylan bisphenylcarbamate | N,N-dimethylacetamide |
| 5. | dextran trisphenylcarbamate | N,N-dimethylacetamide |
| 6. | inulin trisphenylcarbamate | dimethyl sulfoxide |

## Application Example 1

Each of the resolving agents obtained in Examples 1 through 6 was packed in a stainless steel column (25 cm × 0.46 (i.d.) cm) by a slurry filling method. The number of theoretical plates for the compound having a smaller adsorptive power was 4,000 to 7,000.

The high performance liquid chromatograph used was Trirotor-II (a product of Japan Spectroscopic Co.), the detector was UVIDEC-100-III (a product of Japan Spectroscopic Co.), and the polarimeter used was DIP-181 (cell: 5 cm × 0.3 cm (i.d.), a product of Japan Spectroscopic Co.). The flow rate was 0.5 ml/min and the temperature was 25°C.

The results of the resolution are summarized in Tables 1 through 5.

Table 1 (Resolving agent of Example 1)

| Racemates | separation factor α | Eluent |
|---|---|---|
| 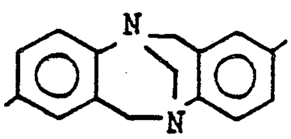 | 1.12 | hexane/2-propanol 99:1 |
| 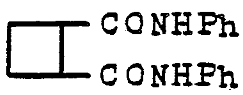 | 1.28 | hexane/2-propanol 90:10 |
| 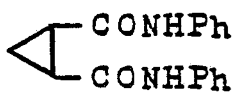 | 1.19 | hexane/2-propanol 90:10 |

Table 2 (Resolving agent of Example 2)

| Racemates | capacity ratio $k_1'$ | separation factor $\alpha$ | Resolution Rs |
|---|---|---|---|
| 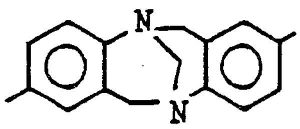 | 0.77(+) | 1.28 | 1.10 |
| $Co(acac)_3$ | 1.80(-) | 1.28 | 0.87 |
| $Cr(acac)_3$ | 1.73(+) | 1.32 | 1.24 |
| 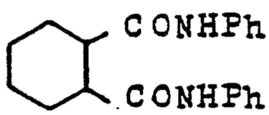 | 0.92(-) | 1.48 | 1.20 |
| 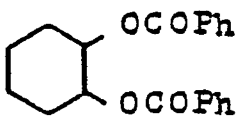 | 0.89(-) | 1.14 | 0.66 |
| 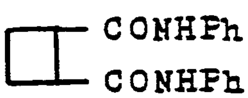 | 1.86(+) | 1.22 | 0.72 |
| 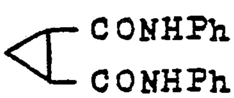 | 1.83(+) | 1.52 | 1.70 |

Eluent: hexane/2-propanol (9:1)

Table 3 (Resolving agent of Example 4)

| Racemates | capacity ratio $k_1'$ | Separation factor α | Resolution Rs |
|---|---|---|---|
| 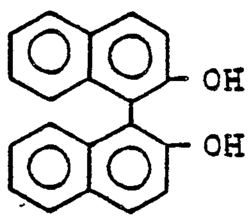 | 5.28(-) | 1.11 | |
| 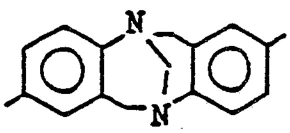 | 0.67(-) | 1.26 | 1.00 |
| $Co(acac)_3$ | 3.27(-) | 1.17 | 0.72 |
| $Cr(acac)_3$ | 2.83(-) | 1.18 | 0.73 |
| 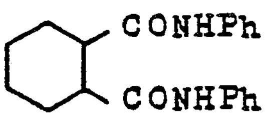 | 0.64(+) | 1.79 | 1.43 |
| 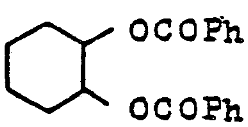 | 3.96(-) | ≈1 | |
| 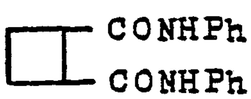 | 1.68(+) | 1.28 | 0.80 |
| 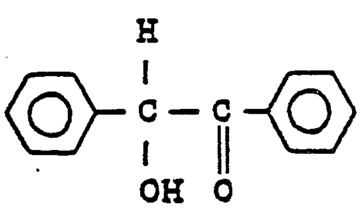 | 2.66(+) | 1.14 | 0.95 |

Eluent: hexane/2-propanol (9:1)

Table 4 (Resolving agent of Example 3)

| Racemates | Separation factor α | Eluent |
|---|---|---|
| 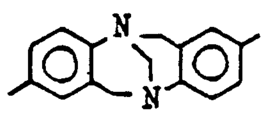 | 1.08 | ethanol/water 70:30 |
| 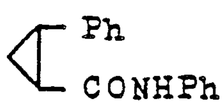 | 1.22 | ethanol/water 80:20 |
| 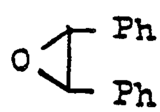 | 1.37 | hexane/2-propanol 90:10 |
| 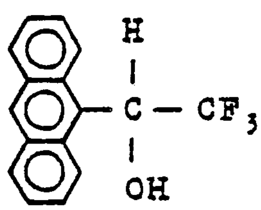 | 1.41 | ethanol/water 70:30 |

Table 5 (Resolution agent of Example 5)

| Racemates | capacity ratio $k'_1$ | Separation factor α | Resolution Rs |
|---|---|---|---|
| 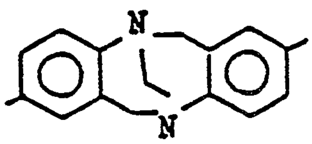 | 0.67(+) | 1.13 | 0.49 |
| $Co(acac)_3$ | 3.39(-) | 2.32 | 2.64 |
| $Cr(acac)_3$ | 2.94(+) | 1.81 | 2.19 |
| 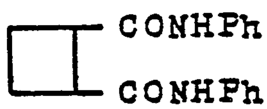 | 0.49(-) | 1.33 | 0.82 |
| 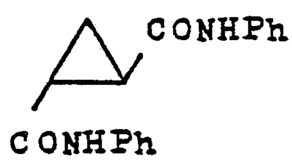 | 0.64(+) | 1.47 | 1.04 |

Eluent: hexane/2-propanol (9:1)

**Claims**

1. A method of separation of optical isomers, geometrical isomers or polymers having different molecular weight ranges from a mixture which comprises treating said mixture with a carbamate derivative of a polysaccharide selected from the group consisting of cellulose, amylose, chitosan, xylan, dextran and inulin, with the proviso that the polysaccharide does not contain a substituent of the formula

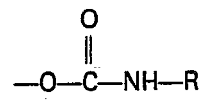

(I)

wherein R represents an atomic group having a nucleus comprising a conjugated π-bond system in which the number of bonds interposed between an atom contained therein and bonded with the amino group and an atom contained in the π-bond system and most distant from said atom is at least 5 even in the shortest route.

2. The method of claim 1, in which said carbamate derivative has a substitution ratio of at least 85 percent.

3. The method of claim 1 or 2, wherein said carbamate derivative has a particle size of 1 μm to 10 mm.

4. Use of the carbamate derivative of a polysaccharide as defined in claim 1 in a chromatographic column or layer.

**Patentansprüche**

1. Verfahren zur Trennung von optischen Isomeren, geometrischen Isomeren oder Polymeren mit verschiedenen Molekulargewichtsbereichen aus einer Mischung, das die Behandlung der Mischung mit einem Carbamatderivat eines Polysaccharids, gewählt aus der Gruppe, bestehend aus Cellulose, Amylose, Chitosan, Xylan, Dextran und Inulin, umfaßt mit der Maßgabe, daß das Polysaccharid keinen Substituenten der Formel

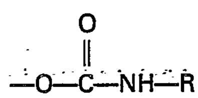

(I)

enthält, worin R eine Atomgruppe mit einem Kern, der ein konjugiertes π-Bindungssystem umfaßt, worin die Zahl der Bindungen zwischen einem darin enthaltenen Atom und gebunden mit der Aminogruppe und einem Atom, enthalten in dem π-Bindungssystem, und am entferntesten von dem Atom, wenigstens 5 auch in der kürzesten Strecke beträgt, darstellt.

2. Verfahren nach Anspruch 1, worin das Carbamatderivat ein Substitutionsverhältnis von wenigtens 85% besitzt.

3. Verfahren nach Anspruch 1 oder 2, worin das Carbamatderivat eine Teilchengröße von 1 μm bis 10 mm besitzt.

4. Verwendung des Carbamatderivats eines Polysaccharids nach Anspruch 1 in einer chromatographischen Säule oder Schicht.

**Revendications**

1. Procédé pour séparer d'un mélange des isomères optiques, des isomères géométriques ou des polymères ayant des plages différentes de poids moléculaires, qui consiste à traiter ledit mélange par un carbamate dérivé d'un polysaccharide choisi dans le groupe formé par la cellulose, l'amylose, le chitosane, le xylane, le dextrane et l'inuline, à condition que le polysaccharide ne contienne pas de substituant de formule

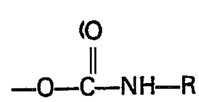

(I)

dans laquelle R représente un groupe atomique ayant un noyau comprenant un système de liaisons π conjuguées dans lequel le nombre de liaisons intercalées entre un atome qu'il contient et qui est lié au groupe amino et un atome contenu dans le système de liaisons π et qui est le plus éloigné dudit atome est d'au moins 5, même par le chemin le plus court.

2. Procédé selon la revendication 1, dans lequel le taux de substitution dudit carbamate dérivé est d'au moins 85 pour cent.

3. Procédé selon la revendication 1 ou 2, dans lequel la grosseur de particules dudit carbamate dérivé est de 1 μm à 10 mm.

4. Utilisation d'un carbamate dérivé d'un polysaccharide tel que défini dans la revendication 1 dans une colonne ou une couche chromatographique.